# EUROPEAN PATENT APPLICATION

(11) **EP 4 144 485 A1**
(43) Date of publication of application: **08.03.2023**
(21) Application number: 22198244.0
(22) Date of filing: 29.11.2012
(51) Int. Cl.: B25J 1/00, A61B 1/005

(54) **POSITIONING DEVICE AND ARTICULATION ASSEMBLY FOR REMOTE POSITIONING OF A TOOL**

(30) Priority: 02.12.2011 US 201161566540 P
(62) Divisional of application: 12852761.1
(71) Applicant: Boston Scientific Scimed, Inc., Maple Grove, MN 55311 (US)
(72) Inventor: PURDY, Craig, A., Campbell, CA 95008 (US); COLE, David, San Mateo, CA 94403 (US); SWOPE, Bretton, San Francisco, CA 94102 (US)
(74) Representative: Vossius & Partner Patentanwälte Rechtsanwälte mbB

(57) **Abstract**

A device for use in guiding the position of a surgical tool from a remote location is disclosed. The device includes a handle, an elongate shaft mounted on the handle, an elongate articulation assembly carried at its proximal end to the shaft and adapted to receive the surgical tool at the assembly's distal end, and a cable operatively connecting the handle to the articulation assembly. The position of the articulation assembly is controlled by rotating a knob on the handle, which moves the articulation into different angled configurations depending on whether the knob is rotated in clockwise or counterclockwise directions.

## Description

The present application claims the benefit of and priority to U.S. Provisional Patent Application Serial No. 61/566,540 filed on December 2, 2011 and is hereby incorporated by reference in its entirety.

### Field of the Invention

The present invention relates to a device and articulation assembly for remote positioning of a tool, such as an intraoral surgical tool.

### Background of the Invention

Many surgical procedures are now done non-invasively, by inserting a surgical tool into a patient's body through a small external incision or through a natural orifice such as the mouth. One of the challenges of non-invasive surgery is to allow the surgeon to easily manipulate the surgical tool, once it is inside the patient's body, to position the tool at a target site where it can be used for performing the desired surgical operation.

A variety of positioning devices for guiding a surgical tool within the body are known. For some types of non-invasive surgery, e.g., arthroscopic surgery, the surgical tool operates close to the point of entry to the surgical site, allowing the surgeon to manipulate the tool position directly close to the surgical site. The gastrointestinal tract is a more challenging environment, since the surgical tool will typically be supported at the distal end of a flexible shaft that may be up to two feet or more in length, for intra-oral access, and because the tool may need to be guided to a region within the GI tract, e.g., the upper portion of the stomach, that is not "in-line" with the shaft carrying the tool.

### Summary of the Invention

The invention includes, in one aspect, an elongate articulation assembly having proximal and distal end regions. The assembly comprises a plurality of links, each mounted on an adjacent link for pivoting with respect to the adjacent link, where a subset of the links have substantially smaller pivot angles, with respect to their adjacent links, in one direction than in the opposite direction, forming an asymmetric section of the assembly. A cable in the assembly extends between the proximal and distal end regions of the assembly, and is operable to urge the assembly links to pivot collectively with respect to one another in a selected clockwise or counterclockwise direction.

In one embodiment, the links forming the asymmetric section of the assembly have pivot angles with respect to their adjacent links of less than 5° in the one direction and pivot angles with respect to their adjacent links of between 10° and 20° or more, e.g., up to 45° in said opposite direction.

The assembly may includes a second section formed by another subset of links, where the links forming the asymmetric section have substantially smaller pivot angles, with respect to their adjacent links, in the one direction than the pivot angles of the links forming the second section, with respect to their adjacent links, in either direction. The two sections can pivot to formed curved sections that lie in substantially the same plane, or in different planes, e.g., orthogonal planes.

In one embodiment, the links forming the asymmetric section of the assembly have pivot angles with respect to their adjacent links of less than 5° in the one direction and pivot angles with respect to their adjacent links of between 10° and 20° or more in the opposite direction, and the links in a second section have pivot angles with respect to their adjacent links of between 10° and 20° or more in either direction. The first and second sections of links may be composed of at least four adjacent links.

The two sections may be separated by an intermediate section whose curvature is substantially unchanged when the links are urged in either the one or the opposite direction. The intermediate section may be formed by three concentric springs, one of which has a helical winding direction opposite that of the other two, and the springs are formed of wires having non-circular cross sections.

Each link in the assembly may have top and bottom cable openings on opposite sides of the link, and the cable includes a first cable arm extending through the top cable opening, and a second cable arm extending through a bottom cable openings. The first and second cable arms may be formed from a single cable looped over the distal end region of the assembly. The assembly may include ferrules positioned between adjacent top or bottom cable openings in the asymmetric section, to limit the extent of pivoting of the links in one direction, where the cable extends through the ferrules between the cable openings in adjacent links.

The links in the assembly may include top and bottom tapered projection plates extending laterally from top and bottom portions of each link, respectively, in the direction facing one of the adjacent links in the assembly, and associated top and bottom plate-receiving slots formed on top and bottom portions of the link, respectively, and facing the other of the adjacent links in the assembly, such that pivoting of a link in either direction moves its top or bottom projection plates into associated top and bottom plate-receiving slots in an adjacent link.

The projection plates on a link may be dimensioned to limit the degree of pivoting of a link in one direction more than in the opposite direction. The top and bottom tapered projection plates extending laterally from top and bottom portions of each link may be curved downwardly and upwardly, respectively, in cross section, on progressing outwardly. The links forming the articulation assembly may be assembled according as described immediately below. Alternatively, the articulation assembly may be formed, for example, as a single-piece molded article, or formed by successive-layer laser printing sintering.

In another aspect, the invention includes an articulation assembly for a medical device comprising a plurality of links that are connected together to form an articulation section. Each of the links includes a link pin received in an associated first pocket of an adjacent link, on one side of the assembly, and a pin opening alignable with a second pin pocket of the same adjacent link, on the other side of the assembly, allowing the two links to be pivotally mounted, one to another, by placing the link pin in the associated first pocket of an adjacent link, aligning the pin opening with the second pocket in the two links, and placing a separate pin through the aligned pin opening and pocket on the other side of links, where the two pins define the pivoting axis of the two links.

The first pin pocket may be beveled to accommodate entry of the first pin on an adjacent link at an angle with respect to said pivoting axis.

The assembly may include other specific features disclosed above. In particular, a subset of the links in the assembly may have substantially smaller pivot angles, with respect to their adjacent links, in one direction than in the opposite direction.

In still another aspect, the invention includes a device for use in guiding the position of a tool, comprising a handle, an articulation assembly operatively coupled at one at one of its ends to the handle and adapted to receive the tool at the assembly's opposite end, and a cable operatively connecting the handle to the articulation assembly. The handle comprises an elongate body defining a central axis, a knob mounted on said body for rotation on the body about said axis, an endless chain mounted within said body for movement in both clockwise and counterclockwise directions in a plane substantially paralleling said axis, and a gear train operatively connecting the knob to said chain, to convert rotational movement of said knob in one direction or the other to a corresponding movement of the chain in a clockwise or counterclockwise direction, respectively.

The cable may be connected to said chain, for movement therewith, by a relief spring, while permitting movement of the cable relative to the chain against a spring force.

The device may further include an elongate shaft by which the articulation assembly is operatively coupled to said handle, and the cable connecting the handle to articulation assembly extends through said shaft. The shaft may include a rigid section adjacent its proximal end and is otherwise flexible along its length.

The endless chain in the handle may include first and second substantially linear regions that move in opposite directions with respect to one another, when the chain is moved in its clockwise or counterclockwise direction, and the cable may include a first and second cable arms that are connected to the first and second chain sections, respectively, for movement therewith, each through a relief spring.

The gear train in the handle may include a ring gear operatively connected to the handle knob for rotation therewith within said body, and a gear assembly that is rotatable about an axis normal to said central axis, and that includes a bevel gear driven by said ring gear, and chain gear that engages said chain. The bevel gear and chain gear may have a selected gear ratio that achieves a desired linear movement of said cable in response to a selected rotational movement of said knob.

The articulation assembly may be movable to different angular configurations substantially within a plane, under the control of the handle, and the plane may be rotated by rotating said handle. In one embodiment, the articulation assembly has a fixed-position distal-end fitting adapted for receiving the tool at a selected one of a plurality of different angular positions with respect to this plane. In another embodiment, the articulation device has a distal-end fitting adapted for receiving said tool at a defined tool orientation, and which further includes an independent handle control and cable mechanism for adjusting the angular position of the fitting.

The handle may include seals to exclude fluids on the outside of the handle from entering the inside of the handle. An exemplary seal is a u cup seal between said knob and said body of device.

These and other objects and features of the invention will become more fully apparent when the following detailed description of the invention is read in conjunction with the accompanying drawings.

### Brief Description of the Drawings

Fig. 1 is a perspective view of a device for use in guiding the position of a surgical tool carried on the distal end of the device within a hollow organ, such as the stomach;
Figs. 2A and 2B illustrate different angular configurations that the articulation assembly in the device can assume when positioning a surgical tool within the stomach;
Fig. 3 is a perspective view of support structure for holding the positioning device next to a patient in a surgical procedure;
Figs. 4A and 4B illustrate a mechanism in the device for placing a surgical tool at a selected orientation;
Fig. 5 is a side, partially cutaway view of an articulation assembly constructed in accordance with the invention;
Fig. 6 shows in perspective view, a portion of a section of the articulation assembly in a curved configuration;
Figs. 7A is a face-on view of an assembly link as viewed from right-to-left in Fig. 6, and with the link rotated 90° in a clockwise direction, and Fig. 7B is a sectional view of the same link taken along section line A-A in Fig. 7A;
Figs. 8A is a face-on view of an assembly link as viewed from right-to-left in Fig. 6, and with the link rotated 27.5° in a clockwise direction, and Fig. 8B is a sectional view of the link taken along section line B-B in Fig. 8A;
Figs. 9A and 9B illustrate, in perspective view, how a pair of adjacent links in the assembly are coupled together for relative pivoting motion; Fig. 9C is a side view of the two links at their maximum pivot angle;
Figs. 10A and 10B show preassembled flexible sections in the assembly of Fig. 5;
Figs. 11A and 11B show an articulation assembly section with cables extending through the links (11A), and the placement of ferrules between adjacent openings on one side of the section links to limit pivoting movement of the links in one direction (11B);
Figs. 12A and 12B are perspective views of an articulation assembly constructed in accordance with another embodiment of the invention;
Figs. 12C and 12D illustrate an articulation assembly formed by successive layer laser sintering;
Fig. 13 is a cutaway perspective view of a handle constructed in accordance with an embodiment of the invention; showing drive components in the handle;
Fig. 14 is a perspective view of the handle's drive components;
Fig. 15 is a perspective view of the interior of the handle seen from the side opposite the drive components;
Fig. 16 illustrates how the endless chain in the handle's drive components are coupled to the assembly cables;
Figs. 17A and 17B show the placement of seals in the handle of the device, and Fig. 17C shows the dynamic U-shaped seals employed in the handle.

### Detailed Description of the Invention

### I. Overview of the positioning device

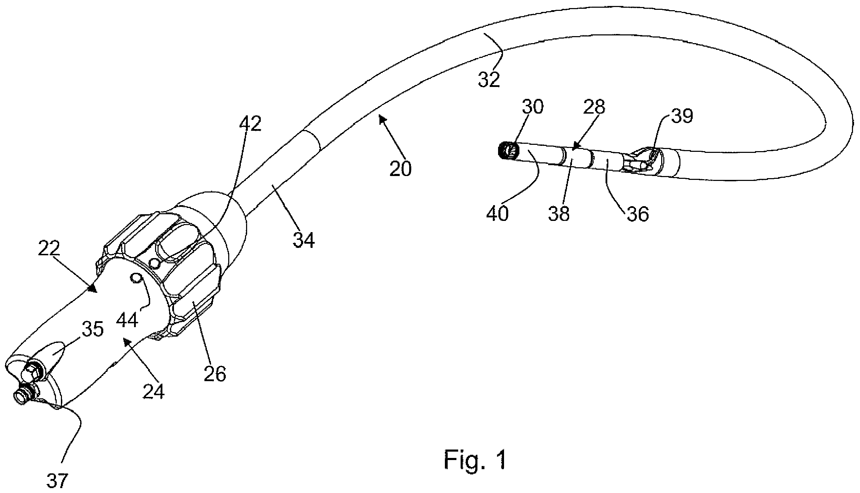

Fig. 1 is a perspective view of a positioning device 20 for use in guiding the position of a tool (not shown) carried on the distal end of the device to a selected location, where the tool can be operated to perform a desired operation. In a specific embodiment described herein, the device is designed for use in a surgical operation within a hollow organ, e.g., a patient's stomach, where the surgical tool is introduced via an overtube (not shown) pre-inserted into the patient's esophagus. More generally, the device may be used in any surgical or non-surgical setting in which a tool carried at the distal end of the device is positioned to perform a task that is remote from the person directing the task.

Device 20 includes a handle 22 having a two-piece housing 24 and an articulation-control knob 26 mounted on the housing for rotation therewith. As will be seen, rotation of knob 26 in a clockwise or counterclockwise direction acts on the an articulation assembly 28 at the opposite end of the device to control the shape, i.e., angular disposition, of the articulation assembly, in turn, to achieve a desired positioning of a surgical tool (not shown) carried at the distal end of the articulation assembly. A tool (not shown) is carried at a distal-end fitting 30 in the device.

In the embodiment described herein, the handle is joined to the articulation assembly through an elongate flexible shaft 32 dimensioned for accessing the patient's stomach intraorally. Shaft 32 has a rigid, proximal-end section 34 used in mounting the device during operation, as described below. Also as shown in Fig. 1, the handle has a fluid port 35 for use in testing the sealing integrity of the device, as determined by the response to a pressurized fluid introduced at port 35. A port 37 in the handle accommodates an endoscope which extends through the handle and shaft and exits at a distal-end opening 39 in the device. The handle may include or be fitted to include additional ports, such as a vacuum port or fluid-pressure port, by which the user can control the operation of the tool, independent of the articulation assembly.

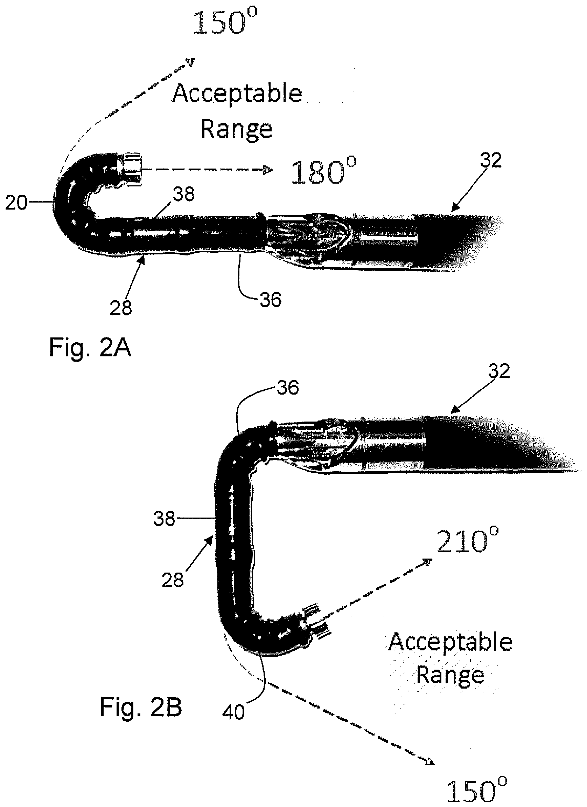

As will be described more fully in Section II below, assembly 28 is formed of a proximal, asymmetric section 36, an intermediate section 38, and a distal section 40 which terminates in a fitting 30. Section 36 retains a relatively straight configuration when the control knob is rotated in one direction, and a curved configuration, e.g., up to a 90° or more curvature, when the knob is rotated in the opposite direction; intermediate section 38 retains its straight configuration independent of the position of the knob; and section 40 assumes a bent configuration in either direction when the knob is rotated clockwise or counterclockwise. The effect of this asymmetric response is seen in Figs. 2A and 2B, which show the extreme conditions of the articulation assembly when the control knob in the device is moved in a fully clockwise (2A) or fully counterclockwise (2B) direction. In the clockwise direction, section 36 remains straight and section 40 curves in a clockwise direction in the figures, forming the J-shaped configuration shown, where an acceptable range of curvature may be between 150° and 180°. When the knob is moved fully counterclockwise, both sections 36 and 40 form curves of 90° or more. With reference to Fig. 1, a pair of indicators 42, 44 on the knob and handle body, respectively, are aligned when the device is in a "neutral" position, i.e., when the articulation assembly is straight.

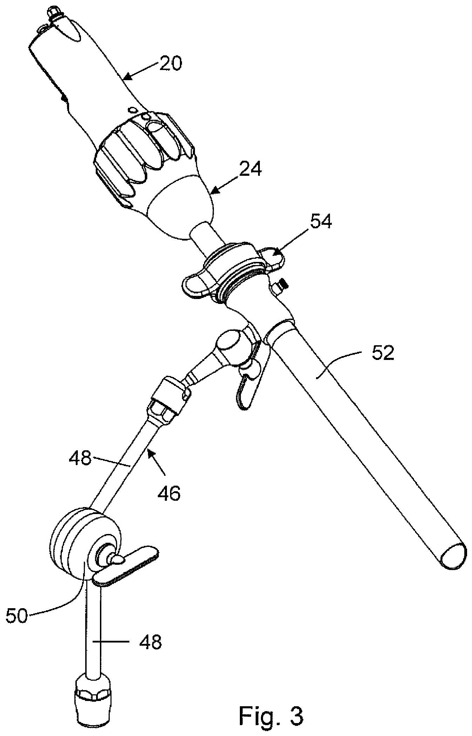

In the surgical setting just mentioned, where a surgeon is performing an operation within a patient's GI tract, the positioning device is preferably mounted an adjustable-position support 46 attached to the surgical table, as shown in Fig. 3. The support has a plurality of support arms, such as arms 48, which are joined together through clamps, such as clamp 50, for positioning a distal-end sleeve 52 at a desired position near the patient. Sleeve 52 is dimensioned to slidably receive rigid section 34 of device 20, for holding the device on the surgical table. A sleeve clamp 54 can be tightened to hold the device at a fixed position in the support, or loosened when it is desired to shift the relative axial or angular positions of the device, for purposes of positioning the surgical tool as will now be described.

For purposes of illustration, it is assumed that the device is being used to perform an operation, e.g., stapling or cutting operation, in a patient's stomach, where the patient is lying on a surgical table, and device 20 is secured on a support 46 after the distal end of the device and attached tool have been guided into the patient's. If the target region within the stomach is close to the gastro-esophageal junction, the surgeon will rotate knob 26 in a clockwise direction to orient the tool in a desired "reverse" direction, as shown in Fig. 2A. Then, by rotating the handle itself to orient the plane containing the curved assembly, and pulling the device in a rearward direction, the surgeon is able to place the attached tool at the target site.

Similarly, the tool can be readily positioned to at a target site remote from the gastro-esophageal junction by rotating the device knob in the opposite direction, causing the articulation assembly to extend in both lateral and axial directions, as illustrate in Fig. 2B, where virtually any angle between 0° and 210° with respect to the long axis of the assembly can be achieved. As above, the surgeon first rotates the handle knob in a counterclockwise direction to achieve a desired two-bend configuration of assembly 28, then rotates the handle itself for proper planar orientation, and moves the device axially until the tool carried on the device is positioned at the target site. Once proper positioning is achieved, the device can be locked into place by tightening clamp 54 on support 46.

The assembly illustrated in Figs. 2A and 2B has two sections whose links pivot to form curves that lie in substantially the same plane. In another embodiment, the assembly sections are oriented with respect such that the links in one section pivot to form a curve in one plane, and the links in the second section pivot to form a curve in another plane, e.g., the two curves lie in orthogonal plane. As will be seen below, the latter embodiment requires that the two sections are oriented such that the pivot axes of the links in one section lie in one plane and the pivot axes of the links in the second section lie in another plane.

An exemplary surgical tool carried on device 20 for positioning with a patient's stomach is a tissue-stapling tool of the type detailed in co-owned U.S. patent applications U.S. 20090125040 and 20100276469, and U.S. Patent Nos. 7,708,181, and 7,909219, all of which are incorporated herein by reference. The tool has proximal and distal stapling members, and a flexible membrane covering the adjoining ends of the two members, forming a tissue chamber therewith. The membrane is provided with an opening through which tissue is drawn into the chamber. In operation, the tool is moved to a selected position within the stomach, with the chamber opening facing the tissue. While vacuum is applied to the chamber, to draw a tissue fold into the chamber, the two members are moved toward one another, causing the arms connecting the two members to spread outwardly, expanding the size of the chamber and thus the size of the tissue fold being formed in the chamber. With a tissue fold is captured in the chamber, and held firmly between the two members, the tool is activated to apply one or more staples across the tissue fold. After releasing the stapled tissue fold, the tool may be withdrawn and reloaded with a new staple cartridge, and the process is then repeated at another selected position in the stomach.

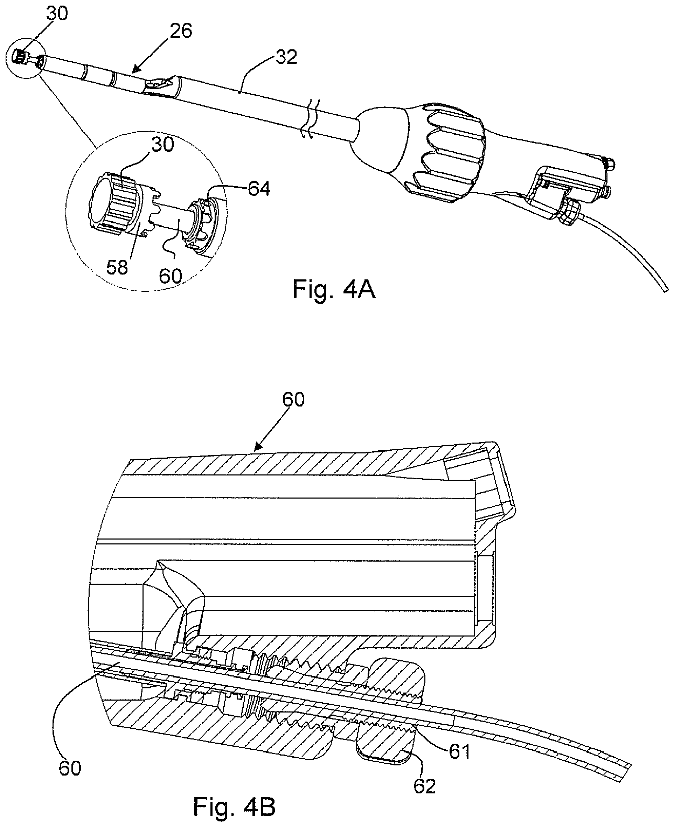

Figs. 4A and 4B illustrate a feature of device 24 for adjusting the angular position of fitting 30, and thus the angular disposition of the tool attached to the fitting. This feature is useful, for example, for orienting the above stapler tool so that its tissue-chamber opening can be placed against the target region, when the tool has been moved to a target tissue region within the stomach. As seen in the figures, fitting 30 and an attached locking wheel 58 are carried at the distal end of a torque cable 60 that extends from the fitting through articulation assembly 28, shaft 32, and handle 24, exiting through a port 61 in a tightening nut 62 threadedly mounted on the handle, for tightening the cable within the device. When the cable is pulled to draw the fitting against the articulation assembly, wheel 62 interlocks with an indexing wheel 64 on the articulation assembly (Fig. 4A) to lock the angular position of the fitting, and the attached tool.

In operation, after moving the plication tool at a selected site within the stomach, the surgeon uses the endoscope to check the orientation of the stapling tool relative to the target tissue. If it is necessary to change the tool orientation, the surgeon loosens nut 62, advances cable 60 to detach the locking and indexing wheels, rotates cable 60 until the desired tool orientation is achieved, pulls the cable to engage wheel 58 with wheel 64, and locks the tool in place by tightening nut 62.

### II. Articulation assembly and its operation

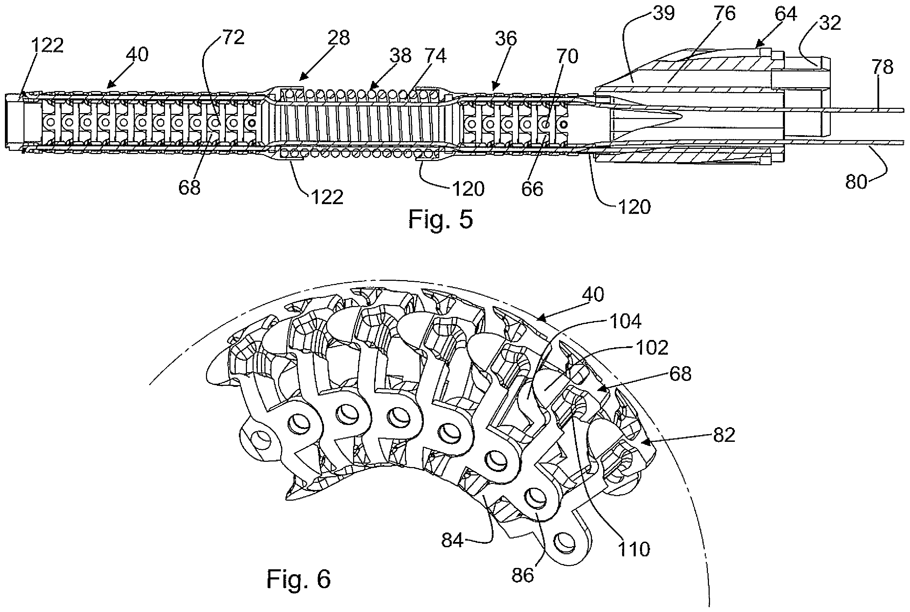

Fig. 5 is an enlarged cross sectional view of articulation assembly 28 in the positioning device described above, where the flexible polymer covering that encases the assembly, seen in Figs. 2A and 2B, has been removed to show the mechanical elements making up the assembly. The figure shows the assembly's proximal, intermediate, and distal sections 36, 38, 40, respectively, and the distal end section 64 of elongate shaft 32 in the positioning device. Sections 36, 40 are each formed of a plurality of links, such as link 66 in section 36 and link 68 in section 40, that are pivotally mounted, one to another, for pivoting about pivot axes normal to the plane of the figure. The pivot axes are shown in the figure as points 70, 72 for links 66, 68, respectively.

In the embodiment shown here, and described below with reference to Figs. 6-11, each link in section 36 is constructed and assembled for pivoting approximately 15° in a downward direction in the figure with respect to its adjacent link, but is substantially constrained from pivoting in the opposite direction, e.g., constrained to a pivot angle less than 5°, preferably less than 2°, with respect to the adjacent link. Thus, when the user adjusts the shape of the assembly by rotating knob 26 in one direction, the six links in the section form a 90° downward curve, as seen in Fig. 2B, while rotation in the opposite direction has little or no effect on the curvature of the proximal section, as seen in Fig. 2A.

Again, with reference to the specific embodiment of the assembly shown, each link in section 40 is constructed and assembled for pivoting approximately 15° in both downward and upward directions in the figure, producing a maximum curvature in the 12-link section of about 180° in both directions, as seen in Figs. 2A and 2B.

Intermediate section 38, which retains its straight configuration independent of sections 36, 40, is formed by three concentric springs, the outermost one of which is shown at 74. One of the three springs has a helical winding direction opposite that of the other two, and at least one of the springs is formed of wires having non-circular cross sections. This construction provides the intermediate section with axial and bending flexibility, but prevents twisting about its long axis when torque forces are applied assembly. It will be appreciated that the articulation assembly of the invention may be composed entirely of pivoting-link sections, without a non-pivoting, intermediate section, or may have one or more such non-pivoting sections, and that the non-pivoting sections, when present, may have a variety of suitable constructions, e.g., a rigid tube or spring or two or more concentric springs.

Completing the description of Fig. 5, a channel 76 extending through the handle and shaft, and terminating at opening 39 in end section 64, accommodates an endoscope used during a surgical procedure. A pair of cables 78, 80 seen in the figure extend from the device handle through the shaft, and are operatively connected to the assembly, for imparting rotational movement in knob 26 to the assembly, as will be described in Section III below. Although not shown here, the shaft may include fluid and control lines extending from the handle, through the shaft and assembly, to the tool carried on the assembly, for activating and controlling the operation of the tool.

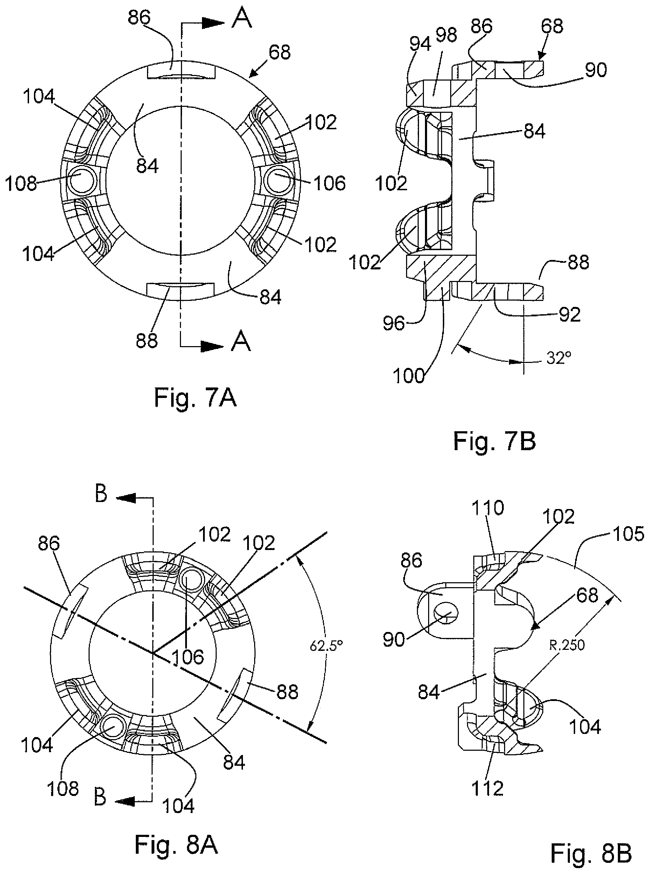

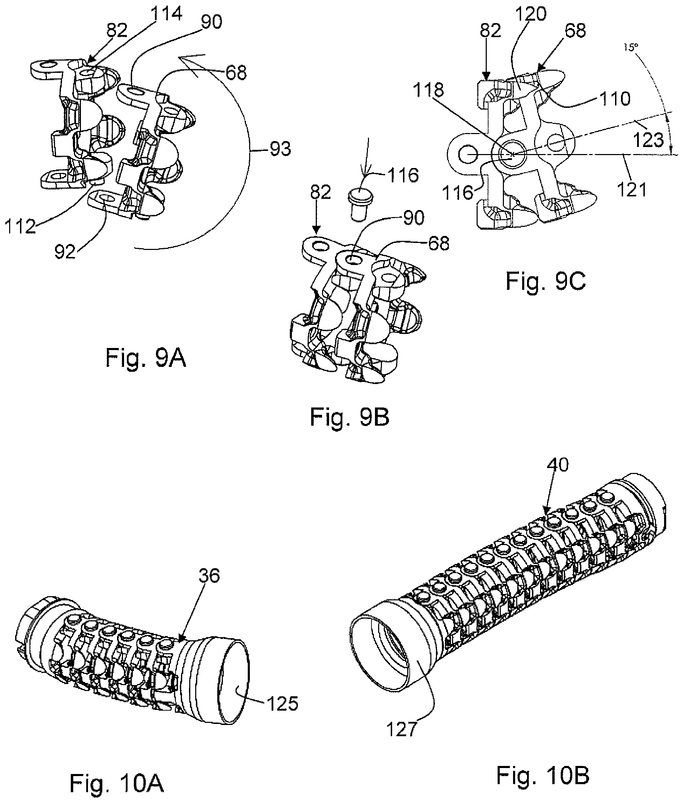

Figs. 6-9 show the construction and link-to-link assembly of the links forming sections 36 and 40. Fig. 6 is a perspective view of a portion of section 40, where each link, such as link 68, is pivoted in a counterclockwise direction about 15° with respect to its adjacent link, such the link 82. Link 68, which is representative, has a unitary construction that includes an annular ring 84 seen best in Figs. 7A and 8A, which are face-on views of link 68 as seen from the right of the link in Fig. 6, and after rotation of the link either 90°.(7A) or 27.5° (8A) in a clockwise direction. The ring supports a pair of assembly brackets 86, 88 which extend rearward (to the right in Figs 6 and 7 and to the left in Fig 8)) from the ring. With reference to Fig. 7B, a cylindrical opening 90 is formed in bracket 86, and a corresponding opening 92 is formed in bracket 88, where opening 92 is flared outwardly about 32° for a purpose to be described. Also as seen in Fig. 7B, assembly brackets 86, 88 in link 66 are joined to ring 84 through top and bottom mounting brackets 94, 96, respectively, which project forwardly (to the left in Figs. 6 and 7, and to the right in Fig. 8) from the ring. Mounting bracket 94 has an opening 98 and mounting bracket 96 has a downwardly projecting pin 100, both for use in assembling the link to an adjacent link, for pivoting with respect thereto, as will be described below with reference to Figs. 9A and 9B.

With continued reference to Figs. 6-8, link 66 has a pair of top and bottom tapered projection plates, such as top projection plates 102 and bottom projections plates 104. As seen in Figs 7A and 7B, the pairs of projections plates are disposed symmetrically between brackets 86, 88, and extend outwardly from the side of ring 84 in the direction opposite that of assembly brackets 86, 88, and in the same direction as mounting brackets 94, 96. As seen best in Fig. 8B, each projection plate, such as plate 102, is tapered inwardly (toward the central axis of the link) on extending outwardly (to the right in Fig. 8B), such that the leading edge of the projection coincides with or lies below the arc of travel 105 of greatest radial dimension of that projection plate. This feature keeps the profile of the projection plates below that of the polymer covering over the assembly, so that the plates don't snag on the covering as the links pivot during the operation of the assembly. Between each pair of projection plates 102 and 104 is an opening 106, 108, respectively, seen Figs. 7A and 8A, which receive cables 78, 80, respectively in the assembly.

Link 66 also includes pairs of top and bottom plate-receiving slots, such as slots 110, 112, respectively seen in Figs. 6 and 8B. The slots are aligned with the corresponding projection plates in the link, and are dimensioned to receive top and bottom projection plates from an adjacent link, respectively, as the "top" or "bottom" side of the adjacent link pivots toward and into the link's plate-receiving slots.

The just-described links forming the articulation assembly may be machined or laser cut from a suitable metal, such as stainless steel or nitinol or other shape-memory metal, or formed by metal injected molding or 3-D printed metal laser sintering, or may be molded or laser cut from a suitable polymer material, all according to known techniques.

Figs. 9A and 9B illustrate how adjacent links are assembled, one to another, in forming the pivoting link sections in the assembly. Initially, the bottom portions of the two links are coupled by inserting pin 112 in link 82 (corresponding to pin 100 in link 68) into the tapered opening 92 of link 68. As can be appreciated from Fig. 9A, the tapered opening allows the pin to be inserted at a link angle at which the assembly and mounting brackets of the two links can be angularly spaced from one another. Then, with the pin in link 82 received in opening 92 in link 68, the two links are swung together, in the direction of arrow 93 in Fig. 9A, to place an opening 114 in link 82 (corresponding to opening 98 in link 68) into alignment with opening 90 in link 68, as shown in Fig. 9B. The final assembly step involves press fitting or welding a rivet 116 through the aligned openings 90, 114 to secure the two links together for pivoting about an axis 118 extending through rivet 166 and pin 112 (Fig. 9C).

Fig. 9C shows the two links pivoted about axis 118 to a maximum pivot angle at which the pair of projection plates, such as plate 120, on link 82 are fully received in the corresponding plate-receiving slots, such as slot 110 in link 68. As indicated above, the projection plates and slots in the present embodiment are dimensioned to permit a pivot angle of about 15°, as measured by the angle formed at the intersection of two lines extending through adjacent pivot-to-bracket lines 121, 123 in the adjacent links, as illustrated in Fig. 9C. It will be appreciated how the plates and/or slots can be dimensioned to allow different pivot angles, e.g., between 0° and 45°, and that the pivot angles on the two sides of the links can be made asymmetric so that, for example, the links are largely constrained against pivoting in one direction, and have a selected pivot angle, e.g., between 10° and 25° on the other side in the opposite direction. Also contemplated is an embodiment in which a linked section has different allowed pivot angle from one link to the next.

Once a pair of links are pivotally joined, the steps described above are repeated from each next-in-line link until a section having a desired plurality of links is formed. Figs. 10A and 10B show sections 36 and 40 after they have been fully assembled, and capped at their confronting, interior ends, with connecting rings, such as ring 125 in section 36 and ring 127 in section 40. The connecting rings are designed to be press fitted into opposite ends of intermediate section 38, to join the three sections forming assembly 28 together, as seen in Fig. 5.

With continued reference to Fig. 5, cable 78 is threaded through the "upper" openings in the links, such as link 66, forming section 36, within the interior of section 38, and through the "upper" openings in the links forming section 40, such as opening 106 in link 68 (Figs. 7A and 8A). Likewise, cable 80 is threaded through the "lower" openings in the links forming section 36, within the interior of section 38, and through the "lower" openings in the links forming section 40, such as opening 108 in link 68 (Figs. 7A and 8A). The cables are secured individually at the distal ends of the assembly. Alternatively, the two cables may be two arms of a single cable that is simply looped over the distal-most link in section 40, from the "upper" to the "lower" openings in that link.

Where the assembly has first and second sections designed to pivot in different planes, the links in the first section have their cable openings disposed in the plane of curvature of that section, and the links in the second section are rotated with respect to the first-section openings so that they are disposed in the plane of curvature of the second section. In this embodiment, the cables connecting the openings in the two sections will form a step pattern along their lengths at the interface between the two sections, or within a non-pivoting section that joins the first and second sections.

As discussed above, at least one section in assembly 28 is constructed or assembled so that the pivot angle between links is substantially greater in one direction than the other. In one embodiment, such asymmetric pivoting is achieved by fashioning the projection plates and/or receiving slots on one side of the links to permit substantially greater pivoting in one direction than the other, as described above.

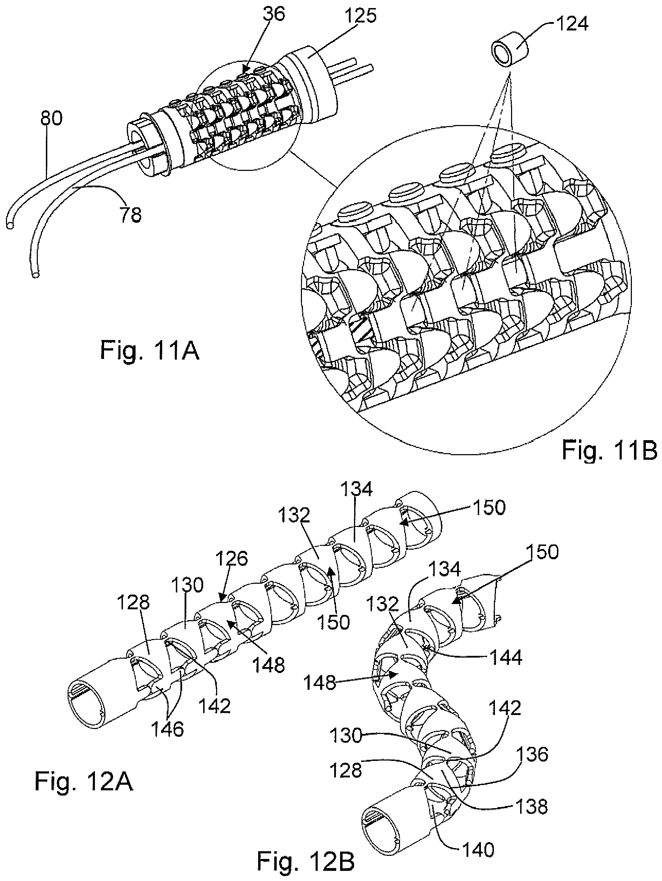

Figs 11A and 11B illustrate a second approach for achieving asymmetric pivoting in a section of the assembly. In this approach, the links forming the assembly are symmetric with respect to their opposed projection plates and receiving slots, and are thus capable of forming an assembly section that can form curved bends in either direction. To achieve asymmetric pivoting, a ferrule 124 is placed between the cable openings in each adjacent pairs of links on one side of the section only, limiting the degree of pivoting allowed between adjacent links on that side of the section only. In the embodiment shown, the ferrules are placed between the link openings corresponding to the upper side of section 36 in Fig. 5, and the associated cable 78 extends through the ferrules between adjacent openings. Typically, the ferrules are dimensioned to prevent any pivoting between links in one direction, i.e., on one side of the section, but shorter ferrules may be employed where some, but not full, pivoting between adjacent links is desired.

Figs. 12A and 12B shows a portion of an articulation assembly 126 constructed as a unitary, single-piece article, in accordance with another embodiment of the invention. The assembly is formed of a rigid, flexible polymer, such as polyethylene, or polymer blend, such as polyethylene/polypropylene, and is formed by laser cutting or polymer molding, according to known methods.

Assembly 126 includes a plurality of links, such as links 128, 130, 132, and 134, which are constructed for pivoting with respect to one another. Each link is composed of a single frame-member ring having upper and lower axially-expanded portions, such as ring 136 in link 128 having upper and lower axially expanded portions 138, 140 respectively (Fig. 12B). The links are joined, one to another, for pivoting relative to an adjacent link, by top and bottom connectors that join the confronting upper and lower ring portions, such as connector 142 joining the upper ring portions in links 128, 130, and connector 144 joining the lower ring portions in links 132, 134.

As with assembly 28 detailed above, a subset of the links in assembly 126 have substantially smaller pivot angles, with respect to their adjacent links, in one direction than in the opposite direction. In the embodiment shown, the left-most four links in the assembly, including links 128, 130, have laterally extending tabs, such as tabs 146 in link 128, that contact the tabs in adjacent links to prevent the links from pivoting along the side of the assembly containing the tabs. Thus, the eight-link assembly shown in the figures has a first section 148 composed of the left-most four links that can pivot in the direction shown in Fig. 12B, but not in the opposite direction, and a second section 150 composed of the right-most links in the figure, including links 132, 134 that can pivot freely in either direction. It will be appreciated that that the assembly can be constructed for a selected degree of pivoting in both sections, either symmetric or asymmetric, by suitable dimensioning of the tabs formed between the links in the assembly. Although not shown here, the links are formed with eyelets or other cable-engaging structure that allows movement-control cables, such as cables 78 80 in Fig. 5, to be threaded along the opposite sides of the links in the "tab" region of the links.

The sections of the assembly may be formed separately, each as a single-piece article, and joined together at their confronting ends or joined at their confronting ends to a non-pivoting intermediate section, as described above. Alternatively, the entire assembly can be formed as a single multi-link article which may include one or more non-pivoting sections formed with the tab configuration shown for section 148, but on both sides of the non-pivoting section, to limit pivoting in either direction.

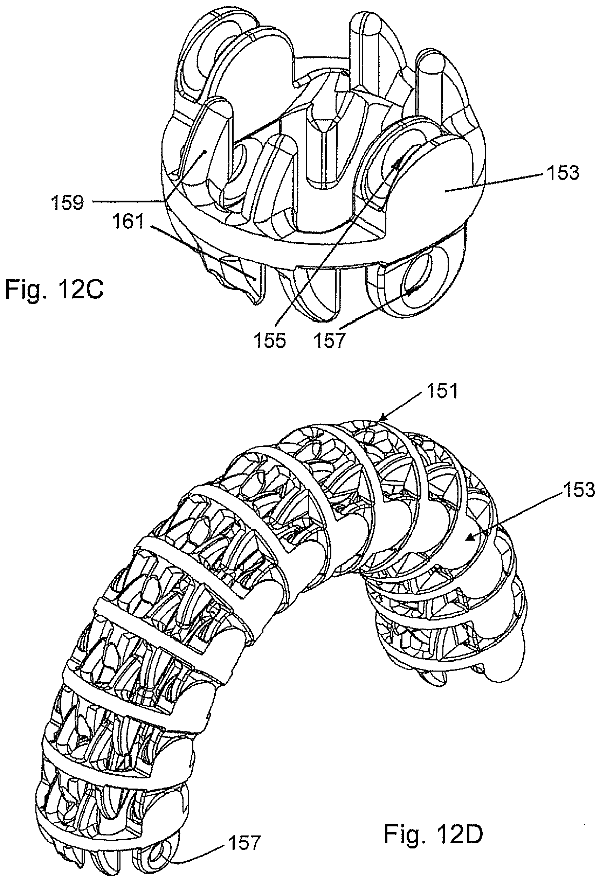

Figs. 12C and 12D show a pivoting section 151 of an articulation assembly constructed, in accordance with another embodiment of the invention, by 3-D printed laser metal sintering. In this technique, a CAD drawing of a fully assembled, pivoting-link assembly is stored in a computer and used to direct a layer-by-layer, link-by-link construction of the assembly, by laying down a thin layer of metal powder and sintering the final desired pattern of that layer by directing a laser beam over the metal-powder layer in the desired pattern.

Fig. 12C is an enlarged perspective view of a single link 153 in the assembly. As seen, the link is designed to include pivot attachment features, such as pin 155 and opening 157 that are built into the unit, allowing link-by-link construction during the assembly manufacture, rather than by subsequent attachment of preformed links. Fingers159, 161 nest in adjacent-link slots to create torque strength in the assembly. Although not seen here, the assembly links have internal stops that interact with barriers on adjacent links to prevent pivoting in the direction opposite that shown in Fig. 12D. It will be appreciated that different sections of the assembly may be designed to pivot freely in both directions, in one direction only, or resist pivoting in either direction. The above method of producing the articulation assembly of the invention by 3-D printed laser metal sintering is another aspect of the invention.

### III. The handle and its operation

The handle in the above positioning device, such as handle 24 in device 20, forms yet another aspect of the invention. As discussed in Section I above, the handle is designed to allow the user, e.g., surgeon, to adjust the position of a remote articulation assembly, asymmetrically, by rotating a handle knob in a selected direction. This, in turn, allows the surgeon to direct a surgical tool for placement at any region of the stomach with a simple one-hand operation.

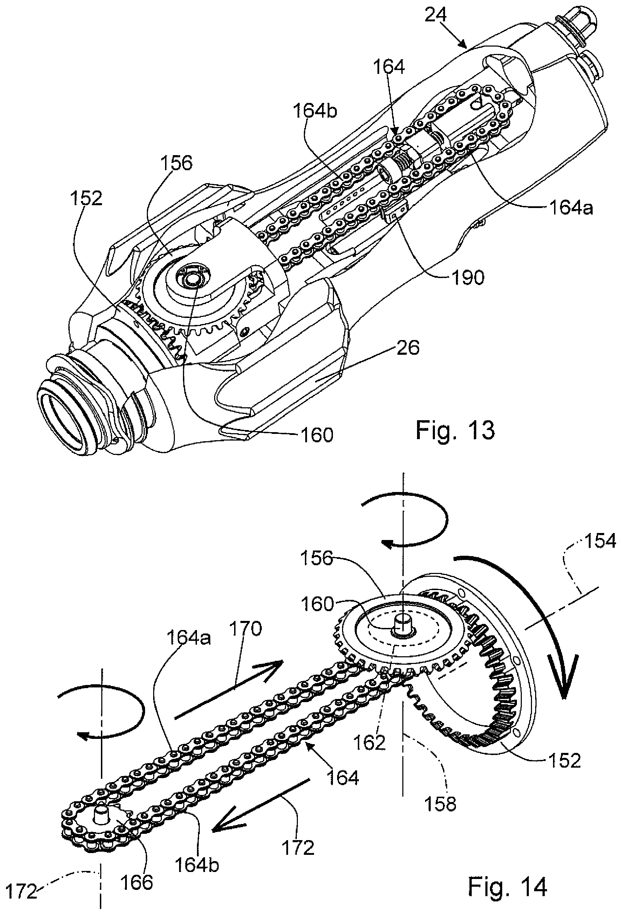

Handle 24 is shown in Fig. 13, with the top portion of the handle cut away to show the mechanical linkage in the handle that converts rotation of knob 26 to movement of an endless chain that, in turn, is connected to the cables that control the position of the remote articulation assembly. Knob 26, which is rotatably mounted on the outside of handle 24, is attached to an internal bevel gear 152 that rotates with the knob about a long axis shown at 154 in Fig. 14. The bevel gear engages a second bevel gear 156 mounted on an axle 160 for rotation about a vertical axis 158 in Fig. 14. Also mounted on this axel, for rotation with gear 160, is a chain-drive gear seen in phantom at 162 in Fig. 14. Gear 162 engages and drives an endless chain 164 that rides between gear 162 and a distal chain gear 166 having a rotational axis 172. The chain may be thought of as having first and second linear arms 164a and 164 b which travel in opposite directions, indicated at 170, 172 in Fig. 14, when the chain is rotated in a clockwise direction, and reverse opposite directions when the knob is rotated in a counterclockwise direction. As will be described below, the two arms of the endless chain are coupled to cables, such as cables 78, 90 in Fig. 5, to control the angular disposition of the articulation assembly according to the angular rotation position of knob 26.

Gears 152, 160, and 162 are also referred to herein, collectively, as a gear train operatively connecting knob 26 to endless chain 164, to convert rotational movement of the knob in one direction or the other to a corresponding movement of the chain in a clockwise or counterclockwise direction.. One advantage of this gear-train configuration is that the disposition of the articulation assembly can be readily controlled by the surgeon rotating knob 26 with one hand, for example, when the positioning device is held in a support. Another advantage is that the ratio of gears 160, 162 can be selected to achieve a desired sensitivity between the degree of rotation in knob 26 and the extent of movement produced in the articulation assembly.

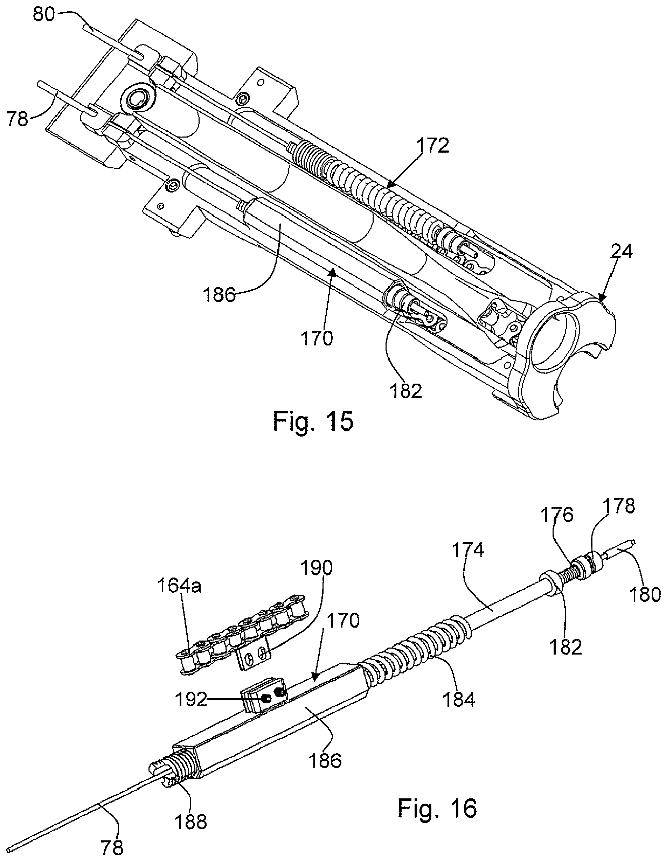

Fig. 15 is a cutaway view of the bottom of the handle, showing the two relief-spring structures 170 and 172 which couple the chain arms 164a, 164b, respectively, to a pair of cables 78, 80, respectively, that are in turn, coupled to the links in the articulation assembly, as described above. Structure 170, which is representative, is shown in partially exploded view in Fig. 16, which also shows a portion of associated chain arm 164a. As seen in this figure, the proximal end portion of cable 78 is housed within a tube 174 having a distal end nut 176 whose head 178 has a central opening (not shown) which stops a nubbin 180 on the distal end of the cable, to hold the cable in the structure when tension is applied to the cable. Tube 174 has a distal-end stop 182 which abuts the distal end of a compression spring 184 in the structure. Spring 184 is carried within a casing 186, and secured in the casing by a distal-end screw 188 on the spring that engages the threaded distal end of the casing. A bracket 190 carried on the chain arm is received in a split bracket 192 on the upper surface of the casing and bolted thereto, to attach the chain arm to the cable casing.

In construction, the two cables are mounted on their corresponding structures in a taut condition, with the cable nubbins pulled against the associated cable tubes, and the tube stops pulled against the associated compression springs, but without tension in the cables. In the "neutral" position of knob 26, the casings holding the two cables are aligned close to the middle region of each chain arm. When knob 26 is moved in a clockwise direction in Fig. 13, chain 164 moves in a clockwise direction, moving chain arm 164a and the attached casing and cable 78 in a rearward or proximal direction, while chain arm 164b and its attached cable 180 move the same distance in a forward direction. That is, rotating knob 26 causes cables 78 and 80 to move an equal distance in opposite directions. When the knob is moved clockwise, cable 78 retracts, pulling the links in the articulation assembly in an upward direction in Figs. 5, ultimately producing the assembly configuration shown in 2A. At the same time, cable 80 extends to accommodate this bending. Similarly, when knob 26 is rotated in a counterclockwise direction, cable 78 is pulled in a proximal direction, while cable 78 extends in a distal direction, causing the links in the articulation assembly to pivot in a downward direction in Fig. 5, ultimately to produce the bend configuration seen in Fig. 2B.

Regardless of the relative position of the cables 78, 80 within housing, the cables are both in a taut condition. Any outside force on the articulation chamber that acts to distort its angular disposition during an operation would therefore cause a stretching force to be applied to one of the two cables. As can be appreciated from Fig. 15, any stretching or tension force applied to either cable, without a corresponding movement of the drive chain in the handle, will be accommodated by a corresponding stretching of the associated relief spring. The relief springs thus act to prevent the cables from being stretched out of shape or breaking if the angular disposition of the articulation assembly is disturbed inadvertently during an operation.

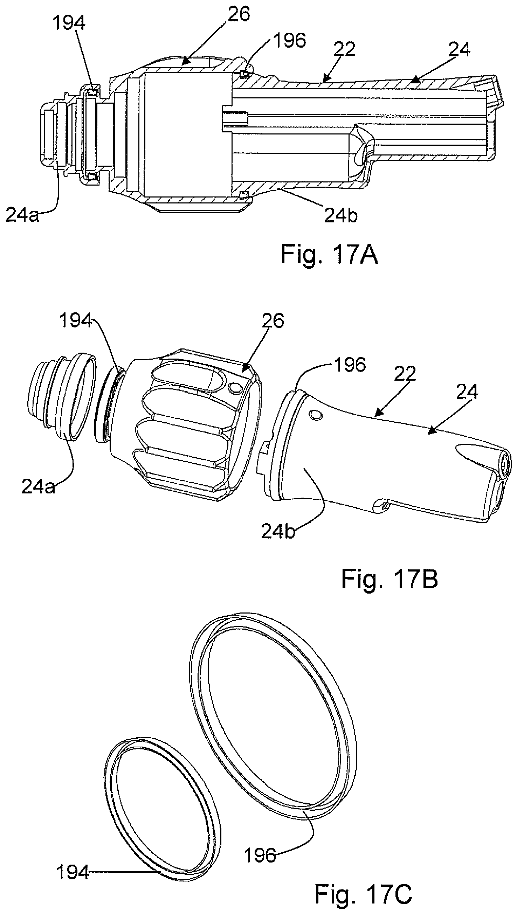

Ideally, the articulation device described is hermetically sealed, allowing it to be reused in a surgical setting without sterilization between uses. Figs. 17A and 17B show the placement of two rotary seals 194 and 196 on the two opposite members 24a and 24b forming the housing in the handle, at the seams between the housing and knob 26. Both seals have the U-cup shape seen in enlarged view in Figs. 17C, and this shape is effective to maintain a good seal at the rotational seams, with a minimum of frictional resistance from the seals when the knob is rotated.

While the invention has been described with respect to specific embodiments, and applications, it will be appreciated that various modification and other applications may be made without departing from the spirit of the invention.

The following aspects are preferred embodiments of the invention
1. An elongate articulation assembly having proximal and distal end regions, comprising
   a plurality of links, each mounted on an adjacent link for pivoting with respect to the adjacent link,
   where a subset of said links have substantially smaller pivot angles, with respect to their adjacent links, in one direction than in the opposite direction, and form an asymmetric section of the assembly, and
   a cable extending between the proximal and distal end regions of the assembly, operable to urge the assembly links to pivot collectively with respect to one another in a selected clockwise or counterclockwise direction.
2. The assembly of aspect 1, wherein the links forming the asymmetric section of the assembly have pivot angles with respect to their adjacent links of less than 5° in said one direction and pivot angles with respect to their adjacent links of between 10° and 20° in said opposite direction.
3. The assembly of aspect 1 which includes a second section formed by another subset of links, where the links forming the asymmetric section have substantially smaller pivot angles, with respect to their adjacent links, in said one direction than the pivot angles of the links forming the second section, with respect to their adjacent links, in either direction.
4. The assembly of aspect 3, wherein the two sections pivot to form curved sections that lie in substantially the same plane.
5. The assembly of aspect3, wherein the two sections pivot to form curved sections that lie in substantially different planes.
6. The assembly of aspect 3, wherein the links forming the asymmetric section of the assembly have pivot angles with respect to their adjacent links of less than 5° in said one direction and pivot angles with respect to their adjacent links of between 10° and 20° in said opposite direction, and the links in the second section have pivot angles with respect to their adjacent links of between 10° and 20° in either direction.
7. The assembly of aspect 3, wherein each section is composed of at least four adjacent links.
8. The assembly of aspect 3, wherein said first and second sections are separated by an intermediate section whose curvature is substantially unchanged when the links are urged in either the one or the opposite direction.
9. The assembly of aspect 8, wherein said intermediate section is formed by three concentric springs, one of which has a helical winding direction opposite that of the other two, and the springs are formed of wires having non-circular cross sections.
10. The assembly of aspect 1, wherein each link has top and bottom cable openings on opposite sides of the link, and said cable includes a first cable arm extending through the top cable openings, and a second cable arm extending through the bottom cable openings.
11. The assembly of aspect 10, wherein said first and second cable arms are formed from a single cable looped over the distal end region of the assembly.
12. The assembly of aspect 10, which includes ferrules positioned between adjacent top or bottom cable openings in the asymmetric assembly section, to limit the extent of pivoting of the links in one direction, where the cable extends through the ferrules between the cable openings in adjacent links.
13. The assembly of aspect 1, wherein said links each include top and bottom tapered projection plates extending laterally from top and bottom portions of each link, respectively, in the direction facing one of the adjacent links in the assembly, and associated top and bottom plate-receiving slots formed on top and bottom portions of the link, respectively, and facing the other of the adjacent links in the assembly, wherein pivoting of a link in either direction moves its top or bottom projection plates into associated top and bottom plate-receiving slots in an adjacent link.
14. The assembly of aspect 13, wherein the projection plates on a link are dimensioned to limit the degree of pivoting of a link in one direction more than in the opposite direction.
15. The assembly of aspect 13, wherein the top and bottom tapered projection plates extending laterally from top and bottom portions of each link are curved downwardly and upwardly, respectively, in cross section, on progressing outwardly.
16. The assembly of aspect 1, wherein each of said links includes a link pin received in an associated first pocket of an adjacent link, on one side of the assembly, and a pin opening alignable with a second pin pocket of the same adjacent link, on the other side of the assembly, allowing the two links to be pivotally mounted, one to another, by placing the link pin in the associated first pocket of an adjacent link, aligning the pin opening with the second pocket in the two links, and placing a second pin through the aligned pin opening and pocket on the other side of links, where the two pins define the pivoting axis of the two links.
17. The assembly of aspect 16, wherein the first pin pocket is beveled to accommodate entry of the first pin on an adjacent link at an angle with respect to said pivoting axis.
18. An articulation assembly for a medical device comprising
   a plurality links that are connected together to form an articulation joint, wherein each of said links includes a link pin received in an associated first pocket of an adjacent link, on one side of the assembly, and a pin opening alignable with a second pin pocket of the same adjacent link, on the other side of the assembly, allowing the two links to be pivotally mounted, one to another, by placing the link pin in the associated first pocket of an adjacent link, aligning the pin opening with the second pocket in the two links, and placing a second pin through the aligned pin opening and pocket on the other side of links, where the two pins define the pivoting axis of the two links.
19. The assembly of aspect 16, wherein the first pin pocket is beveled to accommodate entry of the first pin on an adjacent link at an angle with respect to said pivoting axis.
20. The assembly of aspect 18, wherein a subset of said links have substantially smaller pivot angles, with respect to their adjacent links, in one direction than in the opposite direction.
21. A device for use in guiding the position of a tool, comprising
   a handle,
   an articulation assembly operatively coupled at one at one of its ends to the handle and adapted to receive the tool at the assembly's opposite end, and
      a cable operatively connecting the handle to the articulation assembly,
   said handle comprising
   an elongate body defining a central axis,
   a knob mounted on said body for rotation on the body about said axis,
   an endless chain mounted within said body for movement in both clockwise and counterclockwise directions in a plane substantially paralleling said axis, and
   a gear train operatively connecting the knob to said chain, to convert rotational movement of said knob in one direction or the other to a corresponding movement of the chain in a clockwise or counterclockwise direction, respectively.
22. The device of aspect21, wherein said cable is connected to said chain, for movement therewith, by a relief spring, while permitting movement of the cable relative to the chain against a spring force.
23. The device of aspect 21, which further includes an elongate shaft by which the articulation assembly is operatively coupled to said handle, and the cable connecting the handle to articulation assembly extends through said shaft.
24. The device of aspect 23, wherein said shaft includes a rigid section adjacent its proximal end and is otherwise flexible along its length.
25. The device of aspect 21, wherein said endless chain includes first and second substantially linear regions that move in opposite directions with respect to one another, when the chain is moved in its clockwise or counterclockwise direction, and said cable includes a first and second cable arms that are connected to the first and second chain sections, respectively, for movement therewith, each through a relief spring.
26. The device of aspect 21, wherein said gear train includes a ring gear operatively connected to said knob for rotation therewith within said body, and a gear assembly that is rotatable about an axis normal to said central axis, and that includes a bevel gear driven by said ring gear, and chain gear that engages said chain.
27. The device of aspect 26, wherein said bevel gear and chain gear have a selected gear ratio that achieves a desired linear movement of said cable in response to a selected rotational movement of said knob.
28. The device of aspect 21, wherein the articulation assembly is movable to different angular configurations substantially within a plane, under the control of the handle, and said plane can be rotated by rotating said handle.
29. The device of aspect 21, wherein said articulation assembly has a distal-end fitting adapted for receiving said tool at a selected one of a plurality of different angular positions with respect to said plane.
30. The device of aspect 21, wherein said articulation device has a distal-end fitting adapted for receiving said tool at a defined tool orientation, and which further includes an independent handle control and cable mechanism for adjusting the angular position of said tool.
31. The device of aspect21, wherein has handle includes seals to exclude fluids on the outside of the handle from entering the inside of the handle.
32. The assembly of aspect 31, wherein said seals includes a u cup seal between said knob and said body of device.

## Claims

1. An articulation assembly (28) having proximal and distal end regions, the assembly comprising:
a plurality of links (66), each link mounted on an adjacent link for pivoting with respect to the adjacent link,
wherein a subset of the links (66) have smaller pivot angles, with respect to their adjacent links (66), in one direction than in the opposite direction, and form a proximal section (36) of the assembly (28),
a cable (78, 80) extending between the proximal and distal end regions of the assembly (28), operable to urge the assembly links (66) to pivot collectively with respect to one another, and
a distal-end fitting (30) configured to attach a tool, wherein an angular position of the fitting (30) is adjustable.

2. The assembly of claim 1, wherein the links (66) forming the proximal section (36) of the assembly (28) have pivot angles with respect to their adjacent links (66) of less than 5° in the one direction, and pivot angles with respect to their adjacent links (66) of between 10° and 20° in the opposite direction.

3. The assembly of claim 1 or 2, further comprising a distal section (40) formed by another subset of links (68), wherein the links (66) forming the proximal section (36) have smaller pivot angles, with respect to their adjacent links (66), in the one direction than the pivot angles of the links (68) forming the distal section (40), with respect to their adjacent links (68), in the one direction or the opposite direction,

4. The assembly of claim 3, wherein the proximal section (36) and the distal section (40) pivot to form curved sections that lie in the same plane or in different planes.

5. The assembly of claim 4 or 5, wherein the links (66) forming the proximal section (36) have pivot angles with respect to their adjacent links (66) of less than 5° in the one direction and pivot angles with respect to their adjacent links (66) of between 10° and 20° in the opposite direction, and the links (68) in the distal section (40) have pivot angles with respect to their adjacent links (68) of between 10° and 20° in the one direction or the opposite direction.

6. The assembly of any one of claims 3-5, wherein each of the proximal section (36) and the distal section (40) comprises at least four adjacent links (66, 68).

7. The assembly of any one of claims 3-6, wherein the proximal and distal sections (36, 40) are separated by an intermediate section (38) whose curvature is substantially unchanged when the links (66, 68) are urged in the one direction or the opposite direction.

8. The assembly of claim 7, wherein the intermediate section (38) comprises three concentric springs (74), one of which has a helical winding direction opposite that of the other two, wherein each spring (74) is formed of wires having non-circular cross sections.

9. The assembly of any one of the preceding claims, wherein each link (66) has top and bottom cable openings (106, 108) on opposite sides of the link (66), and the cable (79, 80) includes a first cable arm extending through the top cable openings (106), and a second cable arm extending through the bottom cable openings (108).

10. The assembly of claim 9, wherein:
(i) the first and second cable arms comprise a cable looped over the distal end region, or
(ii) the assembly (28) includes ferrules (124) between adjacent top or bottom cable openings (106, 108) in the proximal section (36), to limit the extent of pivoting of the links (66) in the one direction, wherein the cable (78) extends through the ferrules (124) between the cable openings (106, 108) in adjacent links (66).

11. The assembly of any one of the preceding claims, wherein each link (66) includes projection plates (102, 104) extending laterally from respective portions of each link (66) in a first direction facing one of the adjacent links (66), and associated plate-receiving slots (110, 112) on respective portions of the link (66) facing the other of the adjacent links (66) in an opposite, second direction,
wherein the plate receiving slots (110, 112) are configured to receive the projection plates (102, 104) via pivoting of each link (66) in the first direction or the second direction.

12. The assembly of claim 11, wherein adjacent links (66) pivot about a pivoting axis (118) to a maximum angle when the projection plates (102, 104) are received in associated plate-receiving slots (110, 112), and wherein the projection plates (102, 104) on each link (66) are configured to limit pivoting of a link (66) in the first direction more than in the second direction.

13. The assembly of claim 11 or 12, wherein each of the links (66) has a different maximum angle from one link (66) to the adjacent link (66).

14. The assembly of any one of the preceding claims, wherein a distalmost link of the plurality of links extends along a longitudinal axis, and wherein the distal-end fitting (30) is rotatable about the longitudinal axis such that an angular position of the distal-end fitting is adjustable relative to an angular position of the plurality of links.

15. The assembly of claim 14, wherein the distal-end fitting is movable along the longitudinal axis relative to the distalmost link.
